Europäisches Patentamt

European Patent Office

Office européen des brevets

(19)

(11) Veröffentlichungsnummer: **0 098 843**

**B1**

(12) # EUROPÄISCHE PATENTSCHRIFT

(45) Veröffentlichungstag der Patentschrift:
23.07.86

(21) Anmeldenummer: 83900273.0

(22) Anmeldetag: 12.01.83

(86) Internationale Anmeldenummer:
PCT/EP 83/00005

(87) Internationale Veröffentlichungsnummer:
WO 83/02390 (21.07.83 Gazette 83/17)

(51) Int. Cl.⁴: **A 61 K 7/06**

(54) **MITTEL ZUR BEKÄMPFUNG DER SEBORRHOE DES BEHAARTEN KOPFES.**

(30) Priorität: 20.01.82 DE 3201511

(43) Veröffentlichungstag der Anmeldung:
25.01.84 Patentblatt 84/4

(45) Bekanntmachung des Hinweises auf die Patenterteilung:
23.07.86 Patentblatt 86/30

(84) Benannte Vertragsstaaten:
AT BE CH DE FR GB LI NL SE

(56) Entgegenhaltungen:
DE-A-1 961 152
DE-A-2 137 036
DE-A-2 240 187
DE-A-2 312 091
GB-A-780 801
US-A-3 035 987
US-A-4 052 515

Seifen-Öle-Fette-Wachse, vol. 97, no. 22, 28
October 1971, publ. by Chemische Industrie,
Augsburg (DE), Kluge und Kruse "Eigenschaften
und Verwendung eines neuen C16-Alkohols",
pages 827-831,

(73) Patentinhaber: Henkel Kommanditgesellschaft auf
Aktien, Postfach 1100 Henkelstrasse 67, D-4000
Düsseldorf- Holthausen (DE)

(72) Erfinder: MÖLLER, Hinrich, Schumannstrasse 11,
D-4019 Monheim (DE)
Erfinder: WALLAT, Siegfried, Marie- Curie- Strasse
9, D-4019 Monheim (DE)
Erfinder: HÖFFKES, Horst, Carlo- Schmidt- Strasse
113, D-4000 Düsseldorf- Hellerhof (DE)
Erfinder: GIEDE, Karl, Schlehenweg 12, D-4010
Hilden (DE)

## Beschreibung

Die Erfindung betrifft topische, kosmetische Mittel zur Verbesserung des fettigen und unästhetischen Aussehens der Haare, welche verzweigtkettige Alkanole und Antioxidantien enthalten.

Die moderne Kosmetik ist bemüht, das durch übermäßig starke Absonderung der Talgdrüsen und der Kopfhaut verursachte fettige und wenig ästhetische Aussehen der Haare zu vermindern. Es ist daher vielfach versucht worden, durch geeignete Mittel die Sekretion der Talgdrüsen zu normalisieren, um dem Haar wieder sein gesundes Aussehen zu geben. Es wurden zur Bekämpfung der Seborrhoe des behaarten Kopfes kosmetische Pflegemittel mit Schwefel-, Quecksilber- oder Teerzusatz verwendet. Dabei hat sich gezeigt, daß diese bekannten antiseborrhoischen Zusätze bei längerer Anwendung häufig zu Nebenwirkungen führen, ohne daß wirklich befriedigende Ergebnisse im Hinblick auf Wirksamkeit und anwendungstechnische Eigenschaften erzielt werden konnten. In der DE-OS 2g 26 267 werden zur Normalisierung der Fettabsonderung 3,7,11-Trimethyl-2,6,10-dodecatrien-1-ol-Derivate als Zusatz zu kosmetischen Pflegemitteln beschrieben. Es hat sich jedoch gezeigt, daß diese Verbindungen nur eine sehr geringe antiseborrhoische Wirkung zeigen.

Es ist aus Seifen-Öle-Fette-Wache 97. Jahrgang No. 22/1971, Seiten 827 - 831 bekannt, inen sogenannten "kosmetichen Hexadecylalkohol" in Kosmeika einzuarbeiten. Es handelt sich hierbei um Hexyldecylalkohol, also einem verzweigtkettigen Alkohol. Es wird eine Handcreme beschrieben (1.c., Seite 830), die neben relativ hohen Mengen Hexadecylalkohol (4,00 Gew.-%) und einer Anzahl weiterer üblicher Creme-Bestandteile ein Antioxidans enthält. Diese Creme ist jedoch nicht für die Behandlung des behaarten Kopfes geeignet und dient nicht als Mittel zur Bekämpfung der Seborrhoe. Aufgabe der vorliegenden Erfindung ist es, ein kosmetisches Mittel bereit zu stellen, welches gegenüber den bekannten Präparaten eine verstärkte Wirkung, ohne nachteilige Folgen auf den menschlichen Körper, hat.

Es wurde nun überraschend gefunden, daß durch eine Kombination von verzweigtkettigen Alkanolen mit Antioxidantien hervorragende antiseborrhoische Effekte auch bei sehr geringer Dosierung erreicht werden.

Erfindungsgemäß werden primare $C_{12}$- $C_5$-Alkanole mit einer oder mehreren Verzweigungen im Molekül, welche gegebenenfalls ungesättigt sein können, verwendet. Als sehr gut brauchbar haben sich 2-Octyl - dodecanol, Isooctadecanol, Farnesol und Phytol erwiesen.

Für die erfindungsgemäße Verwendung geeignete Antioxidantien sind: 2,6-Di-tert. butyl-4-methyl-phenol (BHT), 2,6-Di-tert. butyl-4-15 methoxy-phenol (BHA),Tocopherole (DL-o9-Tocopherol, Vitamin E), Gallussäure-ester.

Die genannten Alkanole und Antioxidantien sind im Handel erhältlich. Die erfindungsgemäße sebosuppressive Wirkstoffkombination ist gut haut- und schleinmhautverträglich und kann zur Behandlung von seborrhoischer Kopfhaut und fetten Haaren in allen üblichen Applikationsformen, wie Haarwässern, Haarshampoos, Haarkuren oder Haarspülungen eingesetzt werden. Neben der erfindungsgemäßen Wirkstoffkombination können diese Mittel übliche Träger- und Hilfsstoffe, wie Wasser, organische Lösungsmittel, oberflächenaktive Verbindungen, öle, Fette, Wachse, Duftstoffe, Farbstoffe, Konservierungsmittel u. dgl. enthalten. Die neuen sebosuppressiven Mittel enthalten o,05 - 1 Gew.-% Alkanole und dieselbe Konzentration an Antioxidantien.

Die antiseborrhoische Wirkung der beanspruchten Alkanole in Kombination mit Antioxidantien wurde durch nachfolgend beschriebene Tierversuche näher untersucht.

Als Versuchstiere dienten männliche Wistar-Ratten von 220 bis 230 g Körpergewicht. Beurteilt wurde visuell der Bräunungsgrad auf dem Rücken dér dort geschorenen Ratten. Die Bräunung wird durch das braune Hautoberflächenlipid der Ratten hervorgerufen. Dieser Test geht von der Beobachtung aus, daß junge weibliche Ratten sowie männliche Ratten nach dem Waschen mit Tensidlösung bzw. einem Lipidlösungsmittel und auch männliche Ratten, die systematisch mit östrogen behandelt wurden, nur die normale helle, rosafarbene Haut nach dem Scheren aufweisen; parallel dazu sind aus den abgeschnittenen Haaren nur noch vergleichsweise sehr geringe Lipidmengen zu extrahieren. Zur Beurteilung der Wirksamkeit wurden die Prüfsubstanzen in alkoholischer Lösung jeweils 6 Ratten halbseitig auf das Rückenfell gepinselt. Die andere Seite wurde nur mit dem Lösungsmittel ohne Wirkstoffe behandelt.

Während der Versuchsdauer von 14 Tagen wurde an insgesamt 9 Tagen einmal appliziert. Zur weiteren Kontrolle diente eine Gruppe von 6 Ratten, die völlig unbehandelt blieben. Am Ende des Versuchs wurden die Tiere am Rücken und an den Flanken geschoren und von einem Beurteilerpanel (6 Personen) unabhängig unter Doppelblindbedingungen visuell abgemustert.

## Bewertungsmethoden

Als 1. Kriterium wurde bewertet, ob die Mehrheit der Beurteiler richtig die behandelte Seite erkannt haben, wobei wie folgt differenziert wurde:

Zeichen Anteil der Beurteiler, die eine Wirkung erkannten

+ + 100 %
+ > 50 % -100 %
- < 50 %

Als 2. Kriterium wurde der Unterschied zwischen rechter und linker Seite gewertet, wobei pro Beurteiler und Tier jeweils 1 Punkt zu vergeben war, und zwar in der Weise, daß die

dunklere Seite mit 1 Punkt

h ellere Seite mit 0 Punkte und

bei Gleichheit beide Seiten mit 0,5 Punkte benotet wurden.

Signifikante Differenzen zwischen unbehandelter und behandelter Seite nach der zweiten Bewertungsmethode zeigen die lokale Wirksamkeit einer Substanz an.

Als 3. Kriterium wurden außerdem noch die Intensitätsunterschiede der Brauntöne nach folgender Skala bewertet:

3 Punkte stark braun

2 Punkte mittel braun

1 Punkt schwach braun

0 Punkte keine Braunfärbung.

Nach der dritten Bewertungsmethode werden die Punktsummendifferenzen zwischen den unbehandelten Kontrolltieren und jeweils den behandelten und unbehandelten Seiten der Versuchstiere gebildet, wobei wiederum signifikante Differenzen zwischen Kontrolltieren und der behandelten Seite der Versuchstiere die Wirkung einer Substanz deutlich machen.

Parallel dazu ist in der Regel auch ein deutlicher Unterschied zwischen der unbehandelten und der behandelten Seite der Versuchstiergruppen zu sehen. Dieser ist aber nicht immer so deutlich wie der zwischen Kontrolltieren und behandelter Seite, wofür es verschiedene Gründe geben kann, wie zum Beispiel mechanische Substanzübertragung von einer auf die andere Seite oder Lösungsmitteleinfluß.

Zur Differenzierung der Effekte gemäß Beurteilungsmethode 2 und 3 wurde das folgende Schema verwendet: Zeichen Punktdifferenz

+ + sehr groß (99,9 % Wahrscheinlichkeit)

+ signifikant (95 % Wahrscheinlichkeit)

- (95 % Wahrscheinlichkeit)

Es wurden die Antioxidantien und Alkohole jeweils allein und sodann unter den glelchen Bedingungen die Kombination von Antioxidans und Alkohol auf sebosuppressive Effektivitätuntersucht. Die Ergebnisse sind in der Tabelle 1 zusammengefaßt.

**Prozentuale Sebumreduktion**

Die Sebumreduktion errechnet sich aus der Punktedifferenz in der Weise, daß man den Quotienten aus der Punktedifferenz P und der Punktezahl für die Kontrolgruppe $P_k$ bildet und den erhaltenen Wert in % angibt.

Nachfolgend werden für die erfindungsgemäßen Mittel Rezepturbeispiele gegeben.

**Beispiel 1**

Shampoo für fettendes Haar

$C^{12}$-$C^{14}$-Fettalkoholethosulfat, Na-SalzTEXAPON®N 42,5 Gew.-T.

28 % Waschaktivsubstanz

Kokosfettsäureethanolamid 3,0 Gew.-T.

NaCl 2,0 Gew.-T.

$Na_2SO_4$ 2,0 Gew. -T.

Phytol 0,1. Gew.-T.

DL-$\alpha$-Tocopherol 0,1 Gew.-T.

Parfümöl 0,1 Gew.-T.

Wasser 50,2 Gew.-T.

**Beispiel 2**

Haarkur

Glycerinmono- und distearat (Tegin M(R)) 0,7 Gew.-T.

kationisches Tensid 2,0 Gew.-T.

Cholesterin 0,2 Gew.-T.

Sojalecithin 0,3 Gew.-T.

Emulgator (Emulgarde A(R)) 8,0 Gew.-T.

2-Octyl-dodecanol-i (Eutanol G (R)) 0,1 Gew.-T.

B H T 0,2 Gew.-T.

Parfümöl 0,3 Gew.-T.

Wasser, entsalzt 88,2 Gew.-T.

**Tabelle**

| Substanz \ Versuchs-Nr. | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| BHT | 0,2 | 0,1 | — | — | — | — | — | — | 0,1 | 0,1 | — | 0,2 | — | 0,1 |
| DL- $\alpha$-Tocopherol | — | — | 0,1 | — | — | — | — | — | — | — | 0,1 | — | 0,1 | — |
| Phytol | — | — | — | 0,1 | — | — | — | — | 0,1 | — | — | — | — | — |
| Farnesol | — | — | — | — | 0,1 | — | — | — | — | 0,1 | 0,1 | — | — | — |
| Eutanol G | — | — | — | — | — | 0,1 | — | — | — | — | — | 0,1 | 0,1 | — |
| Isooctadecanol | — | — | — | — | — | — | 0,1 | — | — | — | — | — | — | 0,1 |
| Bewertungsmethode 1. Kriterium | — | — | — | + | — | — | — | — | + + | + | + | + + | + + | + |
| 2. Kriterium | — | — | — | — | — | — | — | — | + + | + + | + + | + + | + + | + |
| 3. Kriterium | — | — | — | — | — | — | — | — | + + | + + | + + | + + | + + | + + |
| Sebumreduktion (%) | 0 | 0 | 0 | 0 | 0 | 0 | 0 | 0 | 83 | 42 | 33 | 31 | 35 | 28 |

## Patentansprüche

Mittel zur Bekämpfung der Seborrhoe des behaarten Kopfes, gekennzeichnet durch einen Gehalt an mindestens einem gegebenenfalls ungesättigten primären C12 -C25-Alka-von 0,05 - 1,0 Gew.-% und mindestens einem Antioxidans aus der Gruppe 2,6-Di-tert.butyl-4-methyl-phenol(BHT), 2,6-Di-tert.butyl-4-methoxy-phenol (BHA),Tocopherole, Gallusäure-ester, in einer Menge von 0,05-1,0 Gew.-%.

2. Mittel nach Anspruch 1, dadurch gekennzeichnet, daß mindestens ein Alkanol aus der Gruppe 2-Octyl-dodecanol, Isooctadecanol, Farnesol, Phytol enthalten ist.

3. Mittel nach Anspruch 1, dadurch gekennzeichnet, daß mindestens eines der Antioxidantien 2,6-Di-tert.butyl-4-methyl-phenol (BHT), DL-9-Tocopherol oder Vitamin E, enthalten ist.

## Claims

1. A preparation for controlling seborrhoea of the scalp, characterized by a content of at least one optionally unsaturated primary $C_{12}$-$C_{25}$ alkanol with one or more branchings in the molecule in a quantity of from 0.05 to i.0% by weight and at least one antioxidant from the group comprising 2,6-di-tert.-butyl-4-methyl-phenol(BHT), 2,6-di-tert.-butyl-4-methoxyphenol (BHA), tocopherols, gallic acid ester, in a quantity of from 0.05 to 1.0% by weight.

2. A preparation as claimed in Claim 1, characterized in that it contains at least one alkanol from the group comprising 2-octyldodecanol, iso-octadecanol, farnesol, phytol.

3. A preparation as claimed in Claim 1, characterised in that it contains at least one of the antioxidants 2,6-di-tert.-butyl-4-methylphenol (BHT), DL-$\alpha$ tocopherol or vitamen E.

## Revendications

1. Agents pour combattre la séborrhée du cuir chevelu de la tête, caractérisés par une teneur d'au moins un alcanol en $C_{12}$-$C_{25}$ primaire éventuellement insaturé ayant une ou plusieurs ramifications dans la molécule, en une quantité de 0,05 à 1,0% en poids, et d'au moins un antioxydant du groupe s du 2,6-di-t-butyl-4-méthyl-phénol (BHT), du 2,6-di-t-butyl-4-méthoxy-phénol (BHA), des tocophérols, des esters d'acide gallique, en une quantité de 0,05 à 1,0% en poids.

2. Agents selon la revendication 1, caractérisés en ce qu'ils contiennent au moins un alcanol du groupe du 2-octyl-dodécanol, de l'isooctadécanol, du farnésol, du phytol.

3. Agents selon la revendication 1, caractérisés en ce qu'ils contiennent au moins un des antioxydants 2,6-di-t-butyl-4-méthyl-phénol (BHT), DL-$\alpha$tocophérol ou vitamine E.